# FASCICULE DE BREVET EUROPEEN

(11) **EP 2 275 170 B1**
(45) Date de publication et mention de la délivrance du brevet: **29.08.2012**
(21) Numéro de dépôt: 10167037.0
(22) Date de dépôt: 23.06.2010
(51) Int. Cl.: A61N 1/05, A61M 25/00

(54) **Ensemble implantable dans le réseau veineux coronarien, comprenant une sonde de stimulation à vis d'ancrage**
Implantateinheit zum Einsetzen in das Koronarvenennetz, die eine Stimulationssonde mit Verankerungsschraube umfasst
Assembly suitable for implantation in the coronary sinus, including a stimulation probe with anchoring screw

(30) Priorité: 15.07.2009 FR 0954873
(43) Date de publication de la demande: 19.01.2011
(73) Titulaire: Sorin CRM SAS, 92541 Montrouge (FR)
(72) Inventeur: Ollivier, Jean-François, 91190, Villiers le Bacle (FR)
(74) Mandataire: Dupuis-Latour, Dominique

(56) Documents cités:
- EP-A1- 0 993 840
- WO-A1-2005/082445
- WO-A2-02/04062
- US-A- 5 837 007
- US-A1- 2006 122 682

## Description

L'invention concerne les "dispositifs médicaux implantables actifs" tels que définis par la Directive 90/385/CEE du 20 juin 1990 du Conseil des communautés européennes, notamment les implants permettant de surveiller en continu le rythme cardiaque et délivrer si nécessaire au coeur des impulsions électriques de stimulation ou de resynchronisation.

Elle concerne plus précisément les sondes de stimulation cardiaque destinées à être implantées dans le réseau coronarien du coeur pour permettre la stimulation d'une cavité gauche, ventricule ou oreillette.

A la différence des cavités droites pour lesquelles il suffit d'implanter des sondes endocavitaires via le réseau veineux périphérique droit, la mise en place de sondes permanentes dans une cavité du coeur gauche impliquerait des risques opératoires importants, par exemple le risque de passage de bulles vers le réseau vasculaire cérébral situé en aval du ventricule gauche. Pour cette raison, lorsque l'on veut stimuler une cavité gauche, on choisit d'introduire une sonde non pas dans la cavité à stimuler mais dans le réseau coronarien, la sonde étant pourvue d'une électrode qu'il conviendra d'appliquer contre la paroi de l'épicarde et d'orienter vers le ventricule gauche ou l'oreillette gauche, selon le cas.

L'introduction d'une telle sonde se fait par le sinus coronaire débouchant dans l'oreillette droite, donc par voie endocavitaire. Elle est ensuite orientée et poussée le long du réseau des veines coronaires jusqu'au site de stimulation choisi. Cette intervention est particulièrement délicate compte tenu des particularités du réseau veineux et de ses voies d'accès, en particulier le passage de valvules et tortuosités, ainsi que la diminution progressive de diamètre du conduit au fur et à mesure de la progression de la sonde dans la veine coronaire sélectionnée. D'autre part, la position du site de stimulation, le bon contact électrique de l'électrode avec le tissu de l'épicarde, et l'orientation correcte de l'électrode de stimulation sont des aspects particulièrement importants.

Par exemple, dans le cas d'un stimulateur de type "multisite", notamment pour la resynchronisation interventriculaire, les deux points de stimulation ventricule gauche/ventricule droit doivent être les plus éloignés possibles pour optimiser la resynchronisation de l'ensemble des cavités cardiaques. Il est par ailleurs important de réduire les risques de stimulation phrénique pendant ou après la pose.

Ces aspects doivent être évalués non seulement au moment de l'implantation, mais il convient également que la qualité et la précision du contact au site de stimulation ne soient pas sensiblement modifiées à moyen terme, notamment par des variations de posture, des mouvements amples du bras, une forte respiration, etc.

Le EP 0 993 840 A1 (ELA Medical) décrit une sonde préformée adaptée à l'implantation dans le réseau veineux coronarien. La tête de sonde porte une électrode sectorielle et elle présente une double courbure permettant d'assurer une auto-orientation de l'extrémité de sonde de telle sorte que l'électrode sectorielle se trouve dirigée vers l'épicarde lorsque la tête de sonde a atteint son positionnement final. Cette sonde préformée est utilisée en combinaison avec un mandrin qui permet d'en redresser plus ou moins la courbure pendant l'implantation afin de faciliter la progression jusqu'au coeur dans le réseau veineux périphérique, puis la recherche du sinus coronaire et enfin la progression dans le réseau coronarien jusqu'au site de stimulation voulu. Ce type de sonde est commercialisé notamment par la société Sorin sous la référence *Situs LV*.

Les sondes préformées sont parfois difficiles à faire progresser, car elles se déplacent selon le principe de moindre énergie. De plus, leur capacité d'extraction reste inconnue, avec un risque d'endommagement des veines du réseau coronaire lors d'une explantation de la sonde.

Enfin, l'un des inconvénients classiques des sondes préformées est leur tendance à reprendre leur forme initiale (du fait du principe de moindre énergie), qui conduit à des micro-déplacements post-opératoires influant sur la qualité des seuils et/ou la resynchronisation.

Un autre problème réside dans la difficulté qu'il y a à trouver un site de stimulation satisfaisant, à obtenir un bon contact électrique de l'électrode contre le tissu de l'épicarde, et à maintenir ce contact malgré les variations ou sollicitations diverses au cours du temps. Il est en outre essentiel d'éviter toute stimulation phrénique, aussi bien lors de la pose qu'ultérieurement.

Pour remédier à ces difficultés, il a été proposé de disposer plusieurs électrodes le long du corps de sonde pour augmenter les chances d'un compromis acceptable, en donnant éventuellement au corps de sonde une conformation particulière. Le praticien peut ainsi sélectionner, parmi les diverses électrodes présentes sur le corps de sonde, celle procurant la meilleure efficacité sur le plan électrique. D'autre part, pour réduire les risques de variation à moyen terme du contact électrique et de stimulation phrénique ultérieure, les constructeurs ont introduit le concept de "repositionnement électronique", visant à diriger ou rediriger le champ électrique entre différentes électrodes disposées le long de la sonde de stimulation de la cavité gauche et/ou avec une des électrodes de la sonde de stimulation de la cavité droite, ceci afin d'éviter une ré-intervention. La contrepartie de cette solution est une complexité grandissante de la structure de la sonde, la multiplication du nombre d'électrodes entraînant une multiplication du nombre de composants, et donc de liaisons électriques, ou encore le recours à des circuits de multiplexage pour la sélection des diverses électrodes présentes sur une même sonde.

Le WO-A-02/04062 A2 (Medtronic) décrit un technique bien connue d"'approche télescopique" couramment utilisée dans diverses applications (angioplastie notamment) pour canuler soit l'ostium du sinus coronaire soit une veine latérale. Cette technique est basée sur l'utilisation de deux cathéters insérés l'un dans l'autre avec des possibilités de mouvement relatifs longitudinaux et circulaires, ces mouvements étant pilotés par le praticien côté proximal. Il est théoriquement possible d'orienter l'extrémité du cathéter intérieur pour diriger l'extrémité de sa courbure distale vers l'épicarde mais, du fait que dans l'application ici visée le cathéter est "cloisonné/ajusté" dans le réseau veineux, la position d'équilibre recherchée génère beaucoup de contre-couple, qu'il faut combattre (la position recherché est nativement instable).

Or plusieurs phénomènes compliquent la tâche du praticien, notamment le fait que le contrôle fin de l'orientation est assujetti à la qualité de la transmission du couple, elle-même liée en particulier : (i) à la section du cathéter, que l'on souhaite la plus faible possible - donc peu favorable à un bon contrôle - et (ii) à la longueur du cathéter, en l'espèce assez importante, de l'ordre de 70 cm, ici encore défavorable à un bon contrôle. Ce contrôle fin est également très perturbé par les mouvements cardiaques qui rendent l'équilibre de l'ensemble très instable, alors que l'opération de vissage peut nécessiter une dizaine de secondes. Il est à noter que le maintien du cathéter et l'opération de vissage requerront probablement deux opérateurs.

Cette solution est donc très "opérateur dépendante", et dans ces conditions, il semble donc difficile de garantir une mise en place sécurisée de l'hélice d'ancrage.

Le problème à résoudre est d'assurer que l'hélice d'ancrage sera orientée dans la bonne direction lors du vissage. En effet, les qualités de fixation d'une sonde à vis sont largement reconnues dans le domaine des sondes de stimulation cardiaque, mais cette technique n'est pas utilisée à ce jour dans le sinus coronaire car les risques de dissection sont jugés trop importants. La fixation demeure néanmoins le problème principal des sondes de stimulation du ventricule gauche via le sinus coronaire.

Le US 5 837 007 A (Pacesetter) tente de résoudre ce problème par utilisation d'une vis rétractable.

Mais cette solution est complexe, car elle nécessite au minimum trois composants (jusqu'à six composants), avec utilisation d'un élément soluble ou d'un composant dont les caractéristiques mécaniques changent notablement après imprégnation par les fluides corporels. De plus, dans tous les variantes proposées par ce document, la transmission de couple s'effectue via un mandrin engageant directement la vis, ce qui nécessite un accessoire supplémentaire et alourdit le mode opératoire, déjà en lui-même complexe et délicat à mettre en oeuvre.

L'invention vise à remédier aux difficultés ci-dessus, notamment en s'affranchissant des problèmes liés à l'emploi de sondes pourvues d'électrodes multiples.

Le but principal de l'invention est de permettre le choix précis et l'évaluation préalable d'un site, et de garantir ensuite une stabilité optimum et pérenne de l'électrode de stimulation sur ce site.

En effet, si l'on peut s'assurer que l'électrode restera, quoi qu'il advienne, placée au site où elle a été initialement implantée, on évitera les déplacements ultérieurs de la sonde et il ne sera plus nécessaire de pallier les conséquences d'un tel déplacement par des techniques complexes telles que le repositionnement électronique ou la sélection parmi des électrodes multiples.

Essentiellement, l'invention propose d'utiliser pour la sonde de stimulation de la cavité gauche une vis d'ancrage, qui est un mode de fixation bien connu pour les sondes de stimulation des cavités droites.

Les sondes à vis de stimulation des cavités droites sont des sondes endocavitaires qui sont introduites jusqu'au fond du ventricule droit. L'extrémité de la sonde est ensuite poussée jusqu'au fond de la cavité (apex ventriculaire), où elle vient buter, et une rotation de la tête de sonde permet de visser celle-ci dans le tissu du myocarde en assurant une fixation définitive - et réversible.

L'implantation directe d'une telle sonde dans le réseau coronaire par les techniques connues présenterait toutefois des difficultés spécifiques. Tout d'abord, la manipulation d'une sonde à vis fixe ou rétractable dans le sinus créerait des risques élevés, avec des niveaux de dommages très variables pouvant aller jusqu'à la dissection complète du sinus coronaire. D'autre part, une fois la tête de sonde arrivée au niveau du site de stimulation, la vis se trouverait dirigée parallèlement à la direction générale de la veine, donc vers l'aval de celle-ci, et non vers la paroi, alors que l'électrode doit, précisément, être mise en contact étroit avec le tissu de l'épicarde.

Pour résoudre ces deux difficultés (accès sans danger au site souhaité et orientation de la tête de sonde vers l'épicarde), l'invention propose de combiner la sonde à vis avec un outil de pose spécifique assurant une sécurité optimum et procurant un angle de vissage adapté.

De façon caractéristique, cet outil est un cathéter-guide préformé, unique, présentant une double courbure comparable à celle décrite, pour une sonde, par le EP 0 993 840 A1 précité.

On notera toutefois que, dans ce document, la bicourbure est appliquée à la sonde elle-même, car celle-ci est une sonde à électrode sectorielle et il faut faire en sorte que cette électrode soit dirigée vers l'épicarde (des moyens étant en outre prévus pour la maintenir plaquée contre la paroi de la veine à cet endroit).

En revanche, dans la présente invention, la bicourbure est appliquée à l'outil de pose, c'est-à-dire au cathéter-guide seul, avec la conséquence majeure de pouvoir substituer à la sonde sectorielle une sonde à vis d'ancrage, avec tous les avantages propres à ce type de sonde, notamment l'absence de risque de déplacement, l'excellente performance électrique au contact avec les tissus du myocarde, et la facilité d'extraction. Comme on le verra, la solution de l'invention utilise la configuration anatomique locale du sinus coronaire et de ses tributaires (virage à gauche) pour auto-orienter et stabiliser l'extrémité distale de ce cathéter spécifique. L'auto-orientation génèrera localement suffisamment de couple pour forcer l'orientation de la courbure d'appui dans la position voulue, permettant un positionnement précis de la tête de la vis au moment d'entamer son vissage dans les tissus.

Cette solution n'est pas opérateur-dépendante, et il suffit au praticien de pousser le cathéter dans la veine cible pour que celui-ci s'auto-oriente après quelques battements dans la position voulue, libérant ainsi ses deux mains pour l'opération de vissage.

Plus précisément, l'invention propose un ensemble du type général décrit notamment dans le WO 02/04062 A2 précité, correspondant au préambule de la revendication 1. Les éléments propres à l'invention sont exposés dans la partie caractérisante de cette revendication 1. Les sous-revendications visent des modes de réalisation particuliers, avantageux.

On va maintenant décrire un exemple de réalisation de l'invention, en référence aux dessins annexés où les mêmes références numériques désignent d'une figure à l'autre des éléments identiques ou fonctionnellement semblables.
La Figure 1 est une vue en coupe de la tête de sonde de l'ensemble selon l'invention.
La Figure 2 illustre schématiquement la configuration de l'ensemble constitué par la sonde de la Figure 1, combinée à un cathéter-guide à double courbure et à un mandrin introduit dans la sonde, cette illustration étant vue en perspective montrant la forme de l'ensemble dans l'espace.
La Figure 3 est une vue en coupe de la sonde de la Figure 1 après que celle-ci ait été implantée dans une veine cible du réseau coronaire, avec la vis ancrée dans la paroi de l'épicarde.

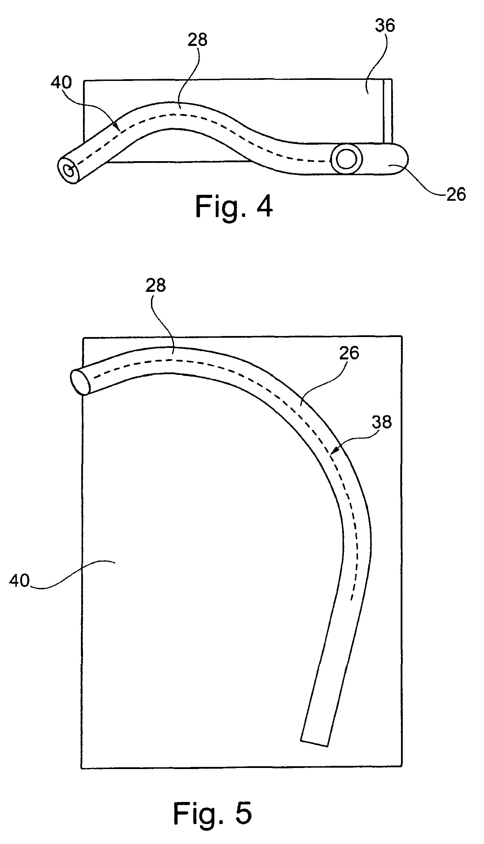

La Figure 4 est une vue de bout selon A-A de la Figure 2.
La Figure 5 est une vue de dessus, selon B-B de la Figure 2.

On va maintenant décrire un exemple de réalisation de l'invention.

Sur les figures, la référence 10 désigne de façon générale la sonde à vis de l'ensemble selon l'invention. Cette sonde 10 comporte un corps de sonde 12 de structure en elle-même connue, généralement une gaine en polyuréthane pour limiter les frottements lorsque la sonde est introduite dans un cathéter-guide, et pour procurer une meilleure sensibilité et une meilleure transmission du couple de torsion. Le diamètre de la gaine du corps de sonde 12 est choisi suffisamment fin pour être compatible avec les cathéters de sous-sélection de veine du réseau coronaire, typiquement un diamètre inférieur ou égal à 5/3 mm (5 French).

La sonde 10 est terminée à son extrémité distale par une vis d'ancrage hélicoïdale 14 en matériau conducteur, reliée par l'intermédiaire d'un embout métallique 16 à un conducteur interne 18 tel qu'un conducteur spiralé assurant la continuité électrique entre la vis d'ancrage, qui fait office d'électrode de recueil et de stimulation, et le générateur situé à l'extrémité proximale (non représentée) de la sonde. On notera que la gaine en polyuréthanne procure une certaine rigidité en torsion au corps de sonde 12, de manière à pouvoir transmettre un couple de torsion depuis l'extrémité proximale jusqu'à l'extrémité distale afin d'entraîner en rotation la vis 14 pour la faire pénétrer par vissage dans le tissu cardiaque.

Le matériau de la vis est avantageusement un alliage NiTi (nitinol), qui présente une capacité de transmission de couple suffisant pour l'application envisagée. L'avantage essentiel de ce matériau est son extrême endurance en fatigue. La contrepartie de cette performance est toutefois la résistance électrique de ce matériau, mais cet inconvénient bien connu peut être compensé par une structure bi-matériau comprenant un coeur en argent (pour la conductivité électrique) enveloppé de nitinol (pour les propriétés de résistance aux contraintes mécaniques), le matériau le moins endurant mécaniquement (argent) étant encapsulé dans la gaine en nitinol.

Pour une stimulation bipolaire, la tête de sonde est en outre pourvue d'une électrode annulaire 20 reliée au générateur par un conducteur distinct (non représenté).

La vis d'ancrage 14 est avantageusement réalisée avec une partie distale 22 formée de spires non jointives, sur une longueur de l'ordre de 1,5 à 2 mm. Cette partie distale 22 est reliée au corps de sonde par l'intermédiaire d'une partie de transition mécanique 24 présentant une souplesse en flexion, par exemple une partie formée de spires jointives en l'absence de sollicitation de la vis.

La raison d'être de cette partie de transition est d'introduire entre la vis proprement dite (la partie qui pénétrera dans les tissus) et le corps de sonde une fonction très élastique afin de limiter l'action mécanique de la partie distale de la sonde sur les tissus cardiaques et la veine.

Cette fonction élastique :
- n'altère pas la transmission de couple entre le corps de sonde et la vis sous les deux aspects de l'efficacité et de la sécurité (effet de carottage) ;
- n'altère pas la transmission de l'impulsion électrique ; et
- est extrêmement endurante aux sollicitions de flexion/compression.

D'autre part, la vis 14 est avantageusement isolée sur toute sa longueur, par exemple par un revêtement de parylène, à l'exception du dernier millimètre distal, qui sera donc la seule partie électriquement active de la vis, ce qui permet de diminuer la surface de stimulation et réduire les risques de stimulation phrénique. Cette partie électriquement active sera en outre enfouie assez profondément dans la paroi de l'épicarde, ce qui permet de concentrer le flux électrique vers le tissu et de stimuler une zone profonde, plus physiologique.

Une vis longue (de l'ordre de 10 à 15 mm) permet de pénétrer profondément la paroi ventriculaire et se rapprocher de la stimulation endocardiaque, grâce à une localisation plus précise du champ électrique garantissant lors de la stimulation une onde de dépolarisation plus rapide de l'endocarde vers l'épicarde.

Enfin, pour éviter le risque de carottage, la lumière interne de la zone flexible de la vis peut être dotée d'une cartouche silicone (éventuellement chargé de stéroïde), pour accentuer l'effet de butée à la transmission du couple.

Dans le mode de réalisation illustré, la vis 14 est une vis active, c'est-à-dire jouant (au moins à son extrémité distale) le rôle d'électrode de stimulation.

En variante, la vis peut être une vis électriquement passive ne servant qu'à l'ancrage de la tête de sonde contre la paroi de l'épicarde. La tête de sonde est dans ce dernier cas pourvue à son extrémité d'une électrode distale par exemple en forme d'anneau de stimulation.

Pour l'implantation de la sonde au site de stimulation choisi on utilisera, de façon caractéristique de l'invention, un cathéter-guide à double courbure.

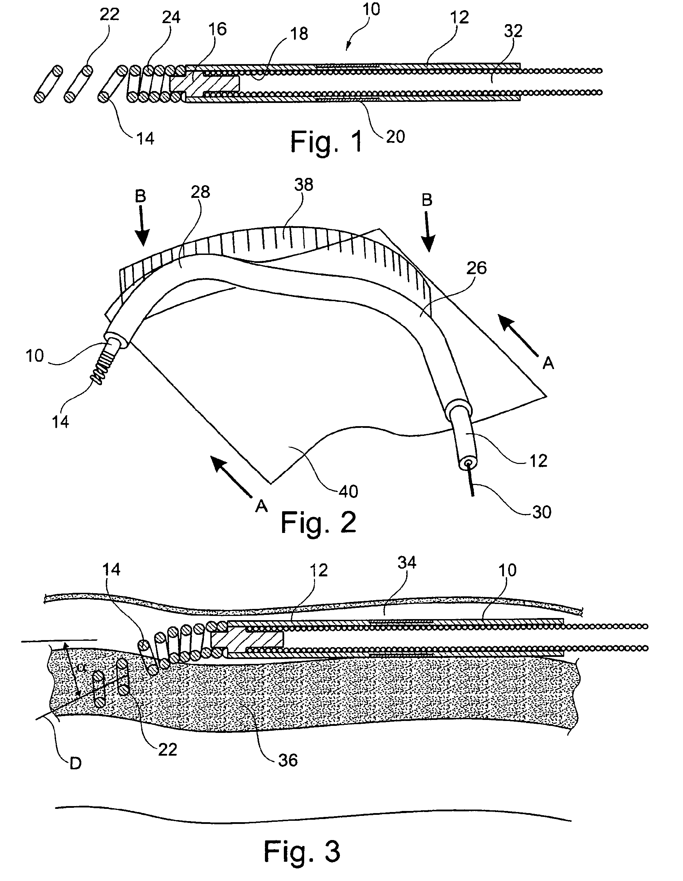

Sur la Figure 2, on a illustré le cathéter-guide 26 avec la sonde 10 introduite à l'intérieur.

La partie distale 28 du cathéter 26 est ouverte à son extrémité, de manière à pouvoir faire émerger l'extrémité distale de la sonde 10 et sa vis d'ancrage 14 par déplacement axial relatif du corps de sonde 12 à l'intérieur du cathéter 26.

En outre, un mandrin 30 est introduit à l'intérieur d'une lumière interne 32 du corps de la sonde 10, de manière à rigidifier celle-ci et redresser la courbure naturelle du cathéter 28 en faisant plus ou moins glisser axialement le mandrin 30 à l'intérieur de la sonde 10.

Comme on l'a indiqué plus haut, le cathéter 26 présente à sa partie distale 28 une double courbure, chacune de ces courbures étant inscrite dans une surface distincte 38, 40. La surface curviligne 38 est une courbure d'orientation qui permet de suivre naturellement la courbure des veines coronaires lors de la progression du cathéter dans le sinus coronaire, tandis que la surface curviligne 40 est une courbure d'appui pou l'orientation de la tête de sonde une fois atteint le site de stimulation. Plus précisément, cette courbure d'appui a pour effet de diriger l'axe de la vis d'ancrage 14 non pas dans le prolongement de la veine cible 34, mais au contraire, comme illustré Figure 3 (après retrait du cathéter) vers la paroi tournée vers l'épicarde 36 de cette veine cible. De cette manière, l'accrochage de la vis et le vissage subséquent de celle-ci seront guidés suivant une direction D faisant un angle α avec la direction générale axiale de la sonde 10, correspondant elle-même approximativement à la direction de progression de la veine cible 34.

On va maintenant décrire la procédure d'implantation de la sonde à vis jusqu'au site choisi.

Cette procédure sera décrite dans son aspect le plus complet, étant entendu que certaines étapes ou l'utilisation de certains éléments peuvent être omis ou adaptés, pour en simplifier le mode opératoire.

On utilise avantageusement la technique dite OTW (*Over-The-Wire*) ou de "filoguidage" consistant à introduire dans le sinus coronaire puis dans le réseau coronaire un fil-guide très fin pourvu à son extrémité distale d'une terminaison très souple, pour ne pas être traumatique. Au préalable, le praticien aura disposé un cathéter principal lui permettant d'accéder au débouché du sinus coronaire, le fil-guide étant ensuite introduit dans ce cathéter puis poussé dans le réseau veineux coronaire.

Le praticien introduit ensuite le cathéter 26 selon l'invention, qui est utilisé comme cathéter de sous-sélection permettant de choisir, sous amplificateur de brillance, le chemin du réseau veineux qui permettra d'atteindre la veine cible correspondant au site de stimulation choisi. L'auto-orientation du cathéter lors de cette phase de positionnement résulte du principe de moindre énergie, notamment lors du "virage à gauche" dans la zone d'intersection de la grande veine cardiaque et d'une veine latérale. L'auto-orientation de la courbure d'orientation génère localement suffisamment de couple pour forcer l'orientation de la courbure d'appui dans la position voulue. La prédominance de l'effet de couple de la courbure d'orientation par rapport à la courbure d'appui est liée au fait que, du point de vue des dimensions :
- le rayon de la courbure d'orientation est supérieur au rayon courbure d'appui ; et
- la longueur de la courbure d'orientation est supérieure à la longueur courbure d'appui).

La distance entre la zone générant le couple d'entraînement (courbure d'orientation) et la zone à maîtriser (courbure d'appui) est donc extrêmement limitée (quelques millimètres).

Il est à noter que ce système fonctionne parce qu'il n'implique qu'un unique cathéter pour fixer les positions relatives des deux courbures.

De ce fait, comme on l'a exposé plus haut, la solution de l'invention n'est pas opérateur-dépendante : il suffit au praticien de pousser le cathéter dans la veine cible pour que le cathéter s'auto-oriente dans la position voulue après quelques battements, libérant ainsi les deux mains du praticien pour l'opération de vissage, tout en garantissant le positionnement précis et sécurisé de la vis et son maintien dans cette position pendant l'étape de vissage qui suivra.

À ce stade, il peut être souhaitable d'introduire un dilatateur creux dans le sous-cathéter afin d'établir une zone de transition progressive entre le fil guide et l'extrémité du sous-cathéter, notamment pour éviter que l'extrémité du cathéter, bien que glissant sur le fil-guide, ne se bloque contre la paroi de la veine à la moindre courbure. Ce dilatateur peut être avantageusement préformé dans sa partie distale pour faciliter la cannulation des veines latérales. Cette option permet de compenser l'auto-positionnement du sous-cathéter vers l'oreillette gauche lors de sa progression dans la grande veine (toujours du fait du principe de moindre énergie, mais mis en oeuvre dans la grande veine cardiaque). Ce comportement donne une opportunité de plus au système, car la solution proposée permet de placer une sonde auriculaire gauche même dans la partie proximale de la grande veine cardiaque.

Une fois le site atteint, le praticien fait coulisser la sonde 10 à l'intérieur du cathéter 26, jusqu'à ce que l'extrémité distale de cette sonde et sa vis d'ancrage 14 émergent de l'extrémité correspondante du cathéter (configuration illustrée Figure 2). Grâce à la double courbure décrite plus haut de l'extrémité distale du cathéter 26, l'orifice distal du cathéter sera dirigé vers l'épicarde 36 en formant un angle α par rapport à la direction générale de la veine cible. Il suffira alors d'introduire dans ce cathéter la sonde 10 jusqu'à ce qu'elle débouche du cathéter pour que sa vis d'ancrage vienne en contact avec l'épicarde. Il sera alors possible de procéder à un *mapping* préalable pour tester électriquement le point de contact et valider le site de stimulation choisi. Si la position n'est pas satisfaisante, il suffira au praticien de déplacer sous contrôle le cathéter le long de la même veine et de tester un nouveau site.

L'ancrage définitif est obtenu en imprimant un mouvement de rotation axiale au corps de sonde (dans le cas d'une sonde à vis fixe) ou à la fiche du connecteur (pour une sonde de type *pin driven*, où côté proximal la fiche de connexion est solidaire d'un conducteur axial s'étendant à l'intérieur du corps de sonde, ce conducteur étant lui-même libre en rotation et relié à la vis d'ancrage à son extrémité distale). Une autre possibilité encore consiste à introduire dans la lumière 32 du corps de sonde un mandrin de vissage spécifique, notamment lorsque la gaine ne présente pas une rigidité en torsion suffisante pour entraîner la vis directement depuis l'extrémité proximale.

En variante, au lieu d'une vis fixe, il est possible d'utiliser une vis rétractable (avec un mécanisme en lui-même connu). Dans ce cas, le mouvement de rotation entraîne tout d'abord le déploiement de la vis hors de son logement, puis la pénétration de celle-ci dans la paroi de l'épicarde.

La dernière étape consiste à retirer le cathéter 26, ce qui est fait selon une procédure classique de découpe au moyen d'un outil de type *slitter* tel que celui décrit dans le EP 2 039 390 A1 (ELA Medical).

La configuration finale, et définitive, est celle illustrée Figure 3.

Parmi les avantages de la technique que l'on vient d'exposer, on peut citer en particulier :
- qualité de la fixation, propre aux vis d'ancrage ;
- stabilité du contact électrique avec les tissus indépendamment de la taille de la veine ;
- possibilité de mapper de larges portions de la veine avant la fixation définitive ;
- possibilité d'élargir la partie exploitable de la veine, notamment vers la zone proximale du réseau veineux, connue pour être la moins exposée aux risques de stimulation phrénique mais qui jusqu'à présent présentait, avec les sondes traditionnelles, l'inconvénient d'une moindre stabilité en raison d'un diamètre plus important ;
- concentration du flux électrique dans une région profonde de l'épicarde, diminuant les risques de stimulation phrénique ;
- excellente capacité d'extraction, par simple dévissage de l'extrémité distale ;
- simplicité mécanique d'ensemble, donc faible coût de fabrication et fiabilité élevée.

## Revendications

1. Un ensemble, implantable dans le réseau veineux coronarien, pour la stimulation d'une cavité cardiaque gauche en combinaison avec un générateur d'un dispositif médical implantable actif, notamment un stimulateur cardiaque ou resynchroniseur, cet ensemble comprenant :
- une sonde de stimulation (10) comportant :
· un corps de sonde avec une gaine (12) en matériau déformable,
· côté distal, une tête de sonde avec au moins une électrode de stimulation apte à venir en contact avec une région, tournée vers l'épicarde (36), de la paroi d'une veine cible (34) du réseau coronaire,
· côté proximal, des moyens de couplage au générateur du dispositif médical ; et
- un cathéter amovible (26) d'implantation de la sonde, ce cathéter comprenant un tube creux ouvert à ses deux extrémités avec une lumière centrale à l'intérieur de laquelle la sonde est apte à être introduite et déplacée en translation à l'intérieur du cathéter, d'abord jusqu'à une position rétractée où la tête de sonde affleure le débouché côté distal de la lumière du cathéter, puis jusqu'à une position déployée où la tête de sonde, avec l'électrode, émerge de ce débouché,
le tube creux du cathéter étant élastiquement déformable et présentant une rigidité relative supérieure à celle de la gaine du corps de sonde,
ensemble dans lequel:
- la sonde (10) est une sonde de type sonde à vis, pourvue d'une vis d'ancrage hélicoïdale saillante (14) prolongeant axialement la tête de sonde, et apte à pénétrer dans le tissu de l'épicarde (36) sous l'effet d'un mouvement de vissage imprimé à la tête de sonde ;
- la gaine (12) du corps de sonde n'est pas préformée à son extrémité distale ; et **caractérisé en ce que** :
- le cathéter (26) comprend un seul tube ; et
- le tube du cathéter (26) est un tube préformé présentant en l'absence de sollicitation deux courbures inscrites dans deux surfaces respectives distinctes (38, 40), ces deux courbures étant aptes à auto-orienter l'extrémité distale du tube de cathéter dans la veine cible (34) avec la tête de sonde en position rétractée, et à maintenir l'axe de la vis d'ancrage en direction de l'épicarde (36) lors d'un mouvement combiné de vissage et de déplacement de la tête de sonde vers la position déployée.

2. L'ensemble de la revendication 1, dans lequel les deux surfaces distinctes (38, 40) définissant les deux courbures respectives comprennent une première surface (38) avec une préforme du tube de cathéter correspondant à une courbure d'orientation et une seconde surface (40) avec une courbure d'appui définie par une forme naturellement coudée de l'extrémité distale du tube du cathéter

3. L'ensemble de la revendication 1, dans lequel la vis d'ancrage (14) est une vis fixe prolongeant axialement la tête de sonde.

4. L'ensemble de la revendication 1, dans lequel la vis d'ancrage est une vis mobile, rétractable dans un logement de la tête de sonde.

5. L'ensemble de la revendication 1, dans lequel la vis d'ancrage (14) est une vis active formant ladite électrode de stimulation.

6. L'ensemble de la revendication 1, dans lequel la vis d'ancrage est une vis passive, électriquement découplée de ladite électrode de stimulation.

7. L'ensemble de la revendication 1, dans lequel la gaine (12) du corps de sonde présente en torsion une rigidité suffisante pour permettre la transmission sur toute sa longueur d'un mouvement de rotation imprimé depuis l'extrémité proximale de la sonde, pour le vissage de la vis d'ancrage.

8. L'ensemble de la revendication 1, dans lequel la gaine du corps de sonde présente en torsion une rigidité insuffisante pour permettre la transmission sur toute sa longueur d'un mouvement de rotation imprimé depuis l'extrémité proximale de la sonde, et dans lequel l'ensemble comprend en outre :
- un mandrin amovible, apte à être introduit dans une lumière (32) du corps de sonde et mobile en translation à l'intérieur de cette lumière jusqu'à la tête de sonde et comportant des moyens de couplage en rotation avec la tête de sonde, ce mandrin présentant en torsion une rigidité suffisante pour permettre la transmission, sur toute sa longueur et jusqu'aux moyens de couplage, d'un mouvement de rotation imprimé depuis l'extrémité proximale du mandrin, pour le vissage de la vis d'ancrage.

9. L'ensemble de la revendication 1, dans lequel le diamètre du corps de sonde est inférieur ou égal à 5/3 mm (5 French).

10. L'ensemble de la revendication 1, dans lequel la vis d'ancrage (14) comprend une partie d'extrémité hélicoïdale (22) apte à pénétrer dans le tissu de l'épicarde, et reliée à la tête de sonde par une partie de transition (24) mécaniquement déformable en flexion.

11. L'ensemble de la revendication 10, dans lequel la partie de transition (24) mécaniquement déformable en flexion est une partie à spires jointives de la vis hélicoïdale.

12. L'ensemble de la revendication 1, dans lequel la vis d'ancrage (14) comprend une partie d'extrémité (22) apte à pénétrer dans le tissu de l'épicarde (36) et comprenant elle-même une région active de pointe électriquement conductrice prolongeant une partie intermédiaire électriquement isolée.

13. L'ensemble de la revendication 12, dans lequel la longueur axiale de la région active de pointe électriquement conductrice est d'au plus 1 mm.

14. L'ensemble de la revendication 1, comprenant en outre :
- un fil-guide amovible (30), ce fil-guide étant apte, après avoir été préalablement introduit dans le réseau veineux coronarien jusqu'à la veine cible, à recevoir le cathéter et à permettre par coulissement le guidage de celui-ci jusqu'à cette veine cible.

## Claims

1. Assembly implantable in the coronary venous system for stimulating a left heart cavity in combination with a generator of an active implantable medical device, in particular a pacemaker or resynchronizer, this assembly comprising:
- a stimulation lead (10) having:
**·** a lead body with a sheath (12) of deformable material,
**·** at the distal end, a lead head with at least one stimulation electrode designed to come into contact with a region, directed towards the epicardium (36), of the wall of a target vein (34) of the coronary system,
**·** at the proximal end, means for coupling to the generator of the medical device; and
- a removable catheter (26) for implanting the lead, this catheter comprising a hollow tube open at both ends and with a central lumen inside which the lead can be introduced and moved in translation inside the catheter, firstly to a retracted position, in which the lead head is flush with the distal mouth of the lumen of the catheter, then to a deployed position, in which the lead head, with the electrode, protrudes from this mouth,
the hollow tube of the catheter being elastically deformable and having a relative rigidity greater than that of the sheath of the lead body,
in which assembly:
- the lead (10) is a screw-type lead, provided with a projecting helical anchoring screw (14) that forms an axial continuation of the lead head, and designed to penetrate the tissue of the epicardium (36) under the effect of a screwing movement imparted to the lead head;
- the sheath (12) of the lead body is not pre-shaped at its distal end; and **characterized in that**:
- the catheter (26) comprises a single tube; and
- the tube of the catheter (26) is a pre-shaped tube which, in the absence of stress, has two curvatures inscribed in two distinct surfaces (38, 40), these two curvatures being designed to permit self-orientation of the distal end of the catheter tube in the target vein (34) with the lead head in the retracted position, and to keep the axis of the anchoring screw in the direction of the epicardium (36) during a combined movement of screwing and displacement of the lead head to the deployed position.

2. Assembly according to Claim 1, in which the two distinct surfaces (38, 40) defining the two respective curvatures comprise a first surface (38), with a preshaping of the catheter tube corresponding to an orientation curvature, and a second surface (40), with a support curvature defined by a naturally bent shape of the distal end of the catheter tube.

3. Assembly according to Claim 1, in which the anchoring screw (14) is a fixed screw forming an axial continuation of the lead head.

4. Assembly according to Claim 1, in which the anchoring screw is a movable screw, retractably mounted in a recess of the lead head.

5. Assembly according to Claim 1, in which the anchoring screw (14) is an active screw forming said stimulation electrode.

6. Assembly according to Claim 1, in which the anchoring screw is a passive screw, electrically decoupled from said stimulation electrode.

7. Assembly according to Claim 1, in which the sheath (12) of the lead body has a torsional rigidity sufficient to permit the transmission, along its entire length, of a rotation movement imparted from the proximal end of the lead, for the purpose of screwing the anchoring screw.

8. Assembly according to Claim 1, in which the sheath of the lead body has a torsional rigidity sufficient to permit the transmission, along its entire length, of a rotation movement imparted from the proximal end of the lead, and in which the assembly further comprises:
- a removable stylet, which can be introduced into a lumen (32) of the lead body, is movable in translation inside this lumen as far as the lead head and has means for coupling in rotation to the lead head, this stylet having a torsional rigidity sufficient to permit the transmission, along its entire length and as far as the coupling means, of a rotation movement imparted from the proximal end of the stylet, for the purpose of screwing the anchoring screw.

9. Assembly according to Claim 1, in which the diameter of the lead body is less than or equal to 5/3 mm (5 French).

10. Assembly according to Claim 1, in which the anchoring screw (14) comprises a helical end part (22), which is designed to penetrate the tissue of the epicardium and is connected to the lead head by a transition part (24) that is mechanically deformable in flexion.

11. Assembly according to Claim 10, in which the transition part (24), mechanically deformable in flexion, is a part of the helical screw with contiguous turns.

12. Assembly according to Claim 1, in which the anchoring screw (14) comprises an end part (22) which is designed to penetrate the tissue of the epicardium (36) and which itself has an active electrically conductive tip region forming a continuation of an electrically insulated intermediate part.

13. Assembly according to Claim 12, in which the axial length of the active electrically conductive tip region is at most 1 mm.

14. Assembly according to Claim 1, further comprising:
- a removable guide wire (30), this guide wire being designed such that, after it has been introduced into the coronary venous system as far as the target vein, it is able to receive the catheter and guide the latter in a sliding movement as far as this target vein.

## Patentansprüche

1. Einheit, die in das Koronarvenennetz zur Stimulation einer linken Herzhöhle in Kombination mit einem Generator einer aktiven implantierbaren medizinischen Vorrichtung, insbesondere eines Herzschrittmachers oder Resynchronisierers, implantierbar ist, wobei diese Einheit Folgendes enthält:
- eine Stimulationssonde (10), die Folgendes aufweist:
· einen Sondenkörper mit einer Hülle (12) aus verformbarem Material,
· auf der distalen Seite einen Sondenkopf mit mindestens einer Stimulationselektrode, die mit einem zum Epikard (36) weisenden Bereich der Wand einer Zielvene (34) des Koronarnetzes in Kontakt kommen kann,
· auf der proximalen Seite Einrichtungen zur Kopplung mit dem Generator der medizinischen Vorrichtung; und
- einen entfernbaren Katheter (26) zur Implantation der Sonde, wobei dieser Katheter ein an seinen beiden Enden offenes Hohlrohr mit einem mittleren Langloch enthält, in dessen Inneres die Sonde eingeführt und im Inneren des Katheters translationsverschoben werden kann, zunächst bis zu einer zurückgezogenen Stellung, in der der Sondenkopf mit der Einmündung auf der distalen Seite des Langlochs des Katheters fluchtet, dann bis zu einer ausgezogenen Stellung, in der der Sondenkopf mit der Elektrode aus dieser Einmündung austritt,
wobei das Hohlrohr des Katheters elastisch verformbar ist und eine relative Steifheit höher als diejenige der Hülle des Sondenkörpers aufweist,
Einheit, bei der:
- die Sonde (10) eine Sonde von der Art Schraubensonde ist, die mit einer vorstehenden Spiralankerschraube (14) versehen ist, die den Sondenkopf axial verlängert, und unter der Wirkung einer dem Sondenkopf verliehenen Schraubbewegung in das Gewebe des Epikards (36) eindringen kann;
- die Hülle (12) des Sondenkörpers an ihrem distalen Ende nicht vorgeformt ist;
und **dadurch gekennzeichnet, dass**:
- der Katheter (26) ein einziges Rohr enthält; und
- das Rohr des Katheters (26) ein vorgeformtes Rohr ist, das in Abwesenheit einer Belastung zwei Krümmungen aufweist, die in zwei unterschiedliche Flächen (38, 40) eingeschrieben sind, wobei diese zwei Krümmungen das distale Ende des Katheterrohrs in der Zielvene (34) mit dem Sondenkopf in zurückgezogener Stellung selbst ausrichten und die Achse der Ankerschraube in Richtung des Epikards (36) bei einer kombinierten Schraubund Verschiebebewegung des Sondenkopfs in die ausgezogene Stellung halten können.

2. Einheit nach Anspruch 1, bei der die zwei unterschiedlichen Flächen (38, 40), die die zwei Krümmungen definieren, eine erste Fläche (38) mit einer Vorform des Katheterrohrs entsprechend einer Ausrichtungskrümmung und eine zweite Fläche (40) mit einer Auflagekrümmung enthalten, die von einer natürlich gebogenen Form des distalen Endes des Katheterrohrs definiert wird.

3. Einheit nach Anspruch 1, bei die Ankerschraube (14) eine ortsfeste Schraube ist, die den Sondenkopf axial verlängert.

4. Einheit nach Anspruch 1, bei der die Ankerschraube eine bewegliche Schraube ist, die in eine Aufnahme des Sondenkopfs zurückziehbar ist.

5. Einheit nach Anspruch 1, bei der die Ankerschraube (14) eine aktive Schraube ist, die die Stimulationselektrode formt.

6. Einheit nach Anspruch 1, bei der die Ankerschraube eine passive Schraube ist, die elektrisch von der Stimulationselektrode entkoppelt ist.

7. Einheit nach Anspruch 1, bei der die Hülle (12) des Sondenkörpers eine ausreichende Torsionssteifigkeit aufweist, um über ihre ganze Länge die Übertragung einer Drehbewegung zu erlauben, die vom proximalen Ende der Sonde ausgehend zum Schrauben der Ankerschraube verliehen wird.

8. Einheit nach Anspruch 1, bei der die Hülle des Sondenkörpers eine unzureichende Torsionssteifigkeit aufweist, um über ihre ganze Länge die Übertragung einer Drehbewegung zu erlauben, die ausgehend vom proximalen Ende der Sonde verliehen wird, und bei der die Einheit außerdem enthält:
- einen entfernbaren Dorn, der in ein Langloch (32) des Sondenkörpers eingeführt werden kann und innerhalb dieses Langlochs bis zum Sondenkopf translationsbeweglich ist und Einrichtungen zur Drehkopplung mit dem Sondenkopf aufweist, wobei dieser Dorn eine ausreichende Torsionssteifigkeit aufweist, um über seine ganze Länge und bis zu den Kopplungseinrichtungen die Übertragung einer Drehbewegung zu erlauben, die vom proximalen Ende des Dorns ausgehend für das Schrauben der Ankerschraube verliehen wird.

9. Einheit nach Anspruch 1, bei der der Durchmesser des Sondenkörpers geringer als oder gleich 5/3 mm (5 French) ist.

10. Einheit nach Anspruch 1, bei der die Ankerschraube (14) einen spiralförmigen Endbereich (22) enthält, der in das Gewebe des Epikards eindringen kann und der mit dem Sondenkopf durch einen mechanisch biegeverformbaren Übergangsbereich (24) verbunden ist.

11. Einheit nach Anspruch 10, bei der der mechanisch biegeverformbare Übergangsbereich (24) ein Teil der Spiralschraube mit aneinander grenzenden Windungen ist.

12. Einheit nach Anspruch 1, bei der die Ankerschraube (14) einen Endbereich (22) enthält, der in das Gewebe des Epikards (36) eindringen kann und selbst einen aktiven Bereich einer elektrisch leitenden Spitze enthält, der einen elektrisch isolierten Zwischenbereich verlängert.

13. Einheit nach Anspruch 12, bei der die axiale Länge des aktiven Bereichs einer elektrisch leitenden Spitze höchstens 1 mm beträgt.

14. Einheit nach Anspruch 1, die außerdem enthält:
- eine entfernbare Drahtführung (30), wobei diese Drahtführung, nachdem sie vorher in das Koronarvenennetz bis zur Zielvene eingeführt wurde, in der Lage ist, den Katheter aufzunehmen und durch Gleiten dessen Führung bis zu dieser Zielvene zu ermöglichen.
